# EUROPEAN PATENT SPECIFICATION

(11) **EP 3 013 943 B1**
(45) Date of publication and mention of the grant of the patent: **10.04.2019**
(21) Application number: 14744961.5
(22) Date of filing: 25.06.2014
(51) Int. Cl.: C12N 5/077, C12N 5/0775

(54) **CELL POPULATIONS HAVING IMMUNOREGULATORY ACTIVITY, METHODS FOR THE PREPARATION AND USES THEREOF**
ZELLPOPULATIONEN MIT IMMUNREGULATORISCHER AKTIVITÄT, VERFAHREN ZUR HERSTELLUNG UND VERWENDUNG DAVON
POPULATIONS CELLULAIRES AYANT UNE ACTIVITÉ IMMUNORÉGULATRICE, PROCÉDÉS DE PRÉPARATION ET UTILISATIONS DE CELLES-CI

(30) Priority: 25.06.2013 GB 201311290; 28.10.2013 GB 201319004
(43) Date of publication of application: 04.05.2016
(73) Proprietor: TiGenix, S.A.U., 28760 Tres Cantos (Madrid) (ES)
(72) Inventor: BRAVO, Eduardo, E-28760 Tres Cantos (Madrid) (ES); PASCUAL, Maria, E-28760 Tres Cantos (Madrid) (ES)
(74) Representative: Carpmaels & Ransford LLP
(86) International application number: PCT/IB2014/062598
(87) International publication number: WO 2014/207679

(56) References cited:
- WO-A1-2009/050282
- WO-A1-2012/123401
- WO-A2-2007/039150
- WO-A2-2008/116157
- ANONYMOUS: "TiGenix reports positive Phase IIa study results in refractory rheumatoid arthritis with allogeneic stem cell product Cx611", Company, Newsroom, Press releases , 22 April 2013 (2013-04-22), XP055143269, Retrieved from the Internet: URL:http://www.tigenix.com/public/uploads/ pdf/en/f5177d809eca202.40307502_TiGenix Cx611 Phase IIa results Final.pdf [retrieved on 2014-09-29]
- Anonymous: "Cx611 in RA", Press Releases, 22 April 2013 (2013-04-22), pages 1-48, XP055144050, WWW Retrieved from the Internet: URL:http://www.tigenix.com/en/download/?s= rNXU8lvo7ZUjEybwArESBX4S%2BkEKDlBvQYuoUTu% 2FQzzXtBQzMAl7TEC3l8DkwthFj%2BYyaSGvZW9cSU 6K10OQDQ%3D%3D [retrieved on 2014-10-02]
- JORGE PAZ RODRIGUEZ ET AL: "Autologous stromal vascular fraction therapy for rheumatoid arthritis: rationale and clinical safety", INTERNATIONAL ARCHIVES OF MEDICINE, BIOMED CENTRAL LTD, LONDON UK, vol. 5, no. 1, 8 February 2012 (2012-02-08), page 5, XP021119262, ISSN: 1755-7682, DOI: 10.1186/1755-7682-5-5
- PHILIPPE BOURIN ET AL: "Stromal cells from the adipose tissue-derived stromal vascular fraction and culture expanded adipose tissue-derived stromal/stem cells: a joint statement of the International Federation for Adipose Therapeutics and Science (IFATS) and the International Society for Cellular Therapy (ISCT)", CYTOTHERAPY, vol. 15, no. 6, 1 June 2013 (2013-06-01), pages 641-648, XP055143289, ISSN: 1465-3249, DOI: 10.1016/j.jcyt.2013.02.006
- ICHIM T E ET AL: "Autologous stromal vascular fraction cells: A tool for facilitating tolerance in rheumatic disease", CELLULAR IMMUNOLOGY, ACADEMIC PRESS, SAN DIEGO, CA, US, vol. 264, no. 1, 1 January 2010 (2010-01-01), pages 7-17, XP027114463, ISSN: 0008-8749 [retrieved on 2010-04-08]
- SCHAEFFLER ANDREAS ET AL: "Concise review: Adipose tissue-derived stromal cells - Basic and clinical implications for novel cell-based therapies", STEM CELLS, ALPHAMED PRESS, DAYTON, OH, US, vol. 25, no. 4, 1 April 2007 (2007-04-01), pages 818-827, XP009133278, ISSN: 1066-5099, DOI: 10.1634/STEMCELLS.2006-0589 [retrieved on 2007-04-01]
- Anonymous: "-TiGenix Press Release 2013", EN -NL) 26 July 2013 -TiGenix Completes EUR 6.5 million Capital Increase, 1 January 2013 (2013-01-01), XP055143981, Retrieved from the Internet: URL:http://www.tigenix.com/en/pages/11/201 3 [retrieved on 2014-10-02]
- OLGA DELAROSA ET AL: "Mesenchymal stem cells as therapeutic agents of inflammatory and autoimmune diseases", CURRENT OPINION IN BIOTECHNOLOGY, vol. 23, no. 6, 1 December 2012 (2012-12-01), pages 978-983, XP55096416, ISSN: 0958-1669, DOI: 10.1016/j.copbio.2012.05.005
- GONZALEZ-REY E ET AL: "Human adipose-derived mesenchymal stem cells reduce inflammatory and T cell responses and induce regulatory T cells in vitro in rheumatoid arthritis", ANNALS OF THE RHEUMATIC DISEASES, BRITISH MEDICAL ASSOCIATION, LONDON, GB, vol. 69, no. 1, 1 January 2010 (2010-01-01), pages 241-248, XP008152922, ISSN: 0003-4967, DOI: 10.1136/ARD.2008.101881

## Description

### BACKGROUND

Rheumatoid arthritis (RA) is an autoimmune disease caused by loss of immunologic self tolerance that leads to chronic inflammation of the joints and subsequent cartilage destruction and bone erosion. The crucial process underlying disease initiation is the induction of autoimmunity to collagen-rich joint components followed by the subsequent events associated with the evolving immune and inflammatory responses. Treating rheumatoid arthritis is a crucial challenge for the health systems of the developed world and increasingly for developing countries. The incidence of rheumatoid arthritis is between 0.5% to 1% of the general population worldwide with little variation of prevalence among countries. It affects over 21 million people worldwide (UN World Population Database, 2004), and has a great impact on the patient's quality of life and gives rise to significant economic and social costs. The economic burden associated to the treatment of RA is formidable for any health care economy and it has been estimated that the combined annual economic cost of this disease is up to €45.5 billion across Europe.

Traditionally, RA has been treated with non-steroidal anti-inflammatory drugs, glucocorticoids and non-biologics-DMARDs. The current pharmacological management of rheumatoid arthritis generally involves early intervention with synthetic disease modifying anti-rheumatic drugs (DMARDs) either singly or in combination. If inflammation cannot be adequately suppressed by these means, typically biologic DMARDs targeting the proinflammatory cytokine TNF are employed. During the last decade, the introduction of biologics has improved therapeutic options for rheumatoid arthritis patients. Biologics, initially directed to neutralize TNF-α, have experienced a big growth over the last number of years regarding both the number of new molecules and the target to which they are directed. Lack of efficacy in some patients, non-tolerability or recurrent secondary infections are factors that have contributed to the need for the development of new therapies. Nonetheless, rheumatoid arthritis remains an unmet clinical need where approximately 20-40% of rheumatoid arthritis patients do not have an adequate response to anti-TNF.

Mesenchymal stem cell therapy has been proposed as an approach for the treatment of inflammatory and autoimmune diseases such as rheumatoid arthritis. Mesenchymal stem cells (MSCs) are non-hematopoietic stromal cells that are able to differentiate into mesenchymal tissues such as bone, cartilage, muscle, ligament, tendon, and adipose. MSCs can be easily isolated from tissues such as bone marrow or adipose tissue and rapidly expanded in culture. MSCs can modulate immune responses, showing antiproliferative and anti-inflammatory capacities which is the basis for their use as potential candidate therapies for the treatment of immune-mediated inflammatory conditions. However there exists a need for establishing effective clinical dosages and dosage regimens for cell therapies in the field of rheumatoid arthritis.

WO2007/039150 teaches that connective tissue derived cells respond to interferon-gamma (IFN-γ) by expressing indolamine-2,3-dioxygenase (IDO) and WO2012/123401 teaches the use of connective tissue derived cells that do not express the cell surface markers CD112 and/or CD155.

WO2018/116157 describes the use of bone marrow derived MSCs to treat gastrointestinal graft- versus-host disease and lung disease, while WO2009/050282 teaches the use of adipose derived MSCs to treat acute colitis in mice.

Rodriguez *et* al. (2012) International Archives of Medicine 5,5 describes the safe administration of adipose SVF (stromal vascular fraction) in patients with rheumatoid arthritis.

Other background publications include (1) a corporate presentation from TiGenix NV of 22nd April 2013 entitled *"Cx611 in RA",* and (2) a press release from TiGenix NV of 22nd April 2013 entitled *"TiGenix report positive Phase IIa study results in refractory rheumatoid arthritis with allogenic stem cell product Cx611"*, which each report positive Phase IIa study results in refractory rheumatoid arthritis with an expanded allogenic adult stem cell product.

### SUMMARY OF THE INVENTION

It has been found that administration of mesenchymal stem cells (MSCs), in particular human adipose tissue derived stromal stem cells (hASCs), may be useful in the treatment of rheumatoid arthritis.

The invention provides a composition comprising a substantially pure population of expanded mesenchymal stem cells (MSCs), wherein the MSCs are allogeneic adipose tissue derived stromal stem cells (ASCs), for use in a method of treating refractory rheumatoid arthritis in a human subject, wherein the composition is intravenously administered to the subject in repeated doses of about 1 million cells/kg at weekly intervals.

In one embodiment, a dose of 1 million mesenchymal stem cells/kg is administered to a human patient at days 1, 8 and 15.These and other aspects of the invention are described in more detail below.

### BRIEF DESCRIPTION OF THE DRAWINGS

Figure 1: Trial scheme. The three doses were administered with a one-week interval each (at days 1, 8, and 15). Patients were followed-up monthly over a six-month period. A total of 46 patients received eASCs.
Figure 2: Percentage of patients in cohorts A (1 million cells/kg), B (2 million cells/kg) and C (4 million cells/kg) reaching ACR20 response at 1, 2 and 3 months after treatment.
Figure 3: Percentage of patients in cohorts A, B and C reaching ACR50 response at 1, 2 and 3 months after treatment.
Figure 4: Percentage of patients in cohorts A, B and C reaching ACR70 response at 1, 2 and 3 months after treatment.
Figure 5: Percentage of patients in cohorts A, B and C reaching DAS28 (ESR) < 3.2 response at 1, 2 and 3 months after treatment. Placebo group is also shown.
Figure 6: Percentage of patients in cohorts A, B and C reaching DAS28 (ESR) < 2.6 response at 1, 2, 3, 4, 5 and 6 months after treatment. Placebo group is also shown.
Figure 7: Percentage of patients in cohorts A, B and C reaching Good + Moderate EULAR response DAS28 (ESR) at 1, 2 and 3 months after treatment. Placebo group is also shown.

### DETAILED DESCRIPTION OF THE INVENTION

### DEFINITIONS

As used herein, the following terms and phrases shall have the meanings set forth below. Unless defined otherwise, all technical and scientific terms used herein have the same meaning as commonly understood to one of ordinary skill in the art to which this invention belongs.

The articles "a" and "an" refer to one or to more than one (i.e., to at least one) of the grammatical object of the article. By way of example, "an element" means one element or more than one element.

The term "about" when used in relation to a value relates to the value ±10%.

By "adipose tissue" is meant any fat tissue. The adipose tissue may be brown or white adipose tissue, derived from, for example, subcutaneous, omental/visceral, mammary, gonadal, or other adipose tissue site. Preferably, the adipose tissue is subcutaneous white adipose tissue. The adipose tissue may comprise a primary cell culture or an immortalized cell line. The adipose tissue may be from any organism having fat tissue. In some embodiments, the adipose tissue is mammalian, and in further embodiments the adipose tissue is human. A convenient source of adipose tissue is liposuction surgery. However, it will be understood that neither the source of adipose tissue nor the method of isolation of adipose tissue is critical to the invention. If cells as described herein are desired for autologous transplantation into a subject, the adipose tissue will be isolated from that subject.

"Adipose tissue-derived stromal stem cells (ASCs)" refers to MSCs that originate from adipose tissue, generally from human adipose tissue (hASCs).

The term "biological medicinal product" shall be taken to mean a protein or nucleic acid-based pharmaceutical substance for therapeutic use, which is typically produced by means other than direct extraction from a native (nonengineered) biological source.

The term "cells/kg" as used herein shall be taken to mean the number of cells (e.g. MSC) administered per kilogram of patient body weight.

The term "constitutively" is understood to mean the expression of a gene without any specific induction.

The term "culture" refers to the growth of cells, organisms, multicellular entities, or tissue in a medium. The term "culturing" refers to any method of achieving such growth, and may comprise multiple steps. The term "further culturing" refers to culturing a cell, organism, multicellular entity, or tissue to a certain stage of growth, then using another culturing method to bring said cell, organism, multicellular entity, or tissue to another stage of growth. A "cell culture" refers to a growth of cells in vitro. In such a culture, the cells proliferate, but they do not organize into tissue per se. A "tissue culture" refers to the maintenance or growth of tissue, e.g., explants of organ primordial or of an adult organ in vitro so as to preserve its architecture and function. A "monolayer culture" refers to a culture in which cells multiply in a suitable medium while mainly attached to each other and to a substrate. Furthermore, a "suspension culture" refers to a culture in which cells multiply while suspended in a suitable medium. Likewise, a "continuous flow culture" refers to the cultivation of cells or explants in a continuous flow of fresh medium to maintain cell growth, e.g. viability. The term "conditioned media" refers to the supernatant, e.g. free of the cultured cells/tissue, resulting after a period of time in contact with the cultured cells such that the media has been altered to include certain paracrine and/or autocrine factors produced by the cells and secreted into the culture. A "confluent culture" is a cell culture in which all the cells are in contact and thus the entire surface of the culture vessel is covered, and implies that the cells have also reached their maximum density, though confluence does not necessarily mean that division will cease or that the population will not increase in size.

The term "culture medium" or "medium" is recognized in the art, and refers generally to any substance or preparation used for the cultivation of living cells. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase that cells growing on a petri dish or other solid or semisolid support are exposed to. The term "medium" also refers to material that is intended for use in a cell culture, even if it has not yet been contacted with cells. In other words, a nutrient rich liquid prepared for bacterial culture is a medium. Similarly, a powder mixture that when mixed with water or other liquid becomes suitable for cell culture may be termed a "powdered medium". "Defined medium" refers to media that are made of chemically defined (usually purified) components. "Defined media" do not contain poorly characterized biological extracts such as yeast extract and beef broth. "Rich medium" includes media that are designed to support growth of most or all viable forms of a particular species. Rich media often include complex biological extracts. A "medium suitable for growth of a high density culture" is any medium that allows a cell culture to reach an OD600 of 3 or greater when other conditions (such as temperature and oxygen transfer rate) permit such growth. The term "basal medium" refers to a medium which promotes the growth of many types of microorganisms which do not require any special nutrient supplements. Most basal media generally comprise four basic chemical groups: amino acids, carbohydrates, inorganic salts, and vitamins. A basal medium generally serves as the basis for a more complex medium, to which supplements such as serum, buffers, growth factors, lipids, and the like are added. Examples of basal media include, but are not limited to, Eagles Basal Medium, Minimum Essential Medium, Dulbecco's Modified Eagle's Medium, Medium 199, Nutrient Mixtures Ham's F-10 and Ham's F-12, McCoy's 5A, Dulbecco's MEM/F-I 2, RPMI 1640, and Iscove's Modified Dulbecco's Medium (IMDM).

The terms "comprise" and "comprising" are used in the inclusive, open sense, meaning that additional elements may be included.

The term "expanded" as used herein when referring to cells shall be taken to have its usual meaning in the art, namely cells that have been proliferated in vitro. A MSC can be expanded to provide a population of cells that retain at least one biological function of the MSC, typically the ability to adhere to a plastic surface, under standard culture conditions. The expanded population of cells may retain the ability to differentiate into one or more cell types.

The term "including" is used herein to mean "including but not limited to". "Including" and "including but not limited to" are used interchangeably.

"Marker" refers to a biological molecule whose presence, concentration, activity, or phosphorylation state may be detected and used to identify the phenotype of a cell.

"Mesenchymal Stem Cells (MSCs)" are multipotent stem cells, *i.e.* they are cells which are capable of giving rise to multiple different types of cells. The term refers to cells which are capable of differentiating into at least one of an osteoblast, a chondrocyte, an adipocyte, or a myocyte. MSCs may be isolated from any type of tissue. The tissue is typically mesenchymal tissue, such as but not limited to bone marrow, periosteum, adipose, dental pulp, skin, spleen, pancreas, ligament, tendon, skeletal muscle, umbilical cord, placenta. Generally MSCs will be isolated from bone marrow, adipose tissue, umbilical cord, or peripheral blood. The MSCs used in the invention may in some embodiments be isolated from bone marrow (BM-MSCs) or adipose tissue (ASCs). In a preferred aspect of the invention, MSCs are obtained from lipoaspirates, themselves obtained from adipose tissue.

A "patient", "subject" or "host" to be treated by the subject method may mean either a human or non-human animal.

The term "pharmaceutical composition" refers to a composition intended for use in therapy. The compositions of the invention are pharmaceutical compositions, intended for use in treating rheumatoid arthritis. The compositions of the invention may include, in addition to MSCs, non-cellular components. Examples of such non-cellular components include but are not limited to cell culture media, which may comprise one or more of proteins, amino acids, nucleic acids, nucleotides, co-enzyme, anti-oxidants and metals.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient.

The term "phenotype" refers to the observable characteristics of a cell, such as size, morphology, protein expression, etc.

The term "progenitor cell" refers to a cell that has the capacity to create progeny that are more differentiated than itself. For example, the term may refer to an undifferentiated cell or cell differentiated to an extent short of final differentiation which is capable of proliferation and giving rise to more progenitor cells having the ability to generate a large number of mother cells that can in turn give rise to differentiated or differentiable daughter cells. In one embodiment, the term progenitor cell refers to a generalized mother cell whose descendants (progeny) specialize, often in different directions, by differentiation, e.g., by acquiring completely individual characters, as occurs in progressive diversification of embryonic cells and tissues. Cellular differentiation is a complex process typically occurring through many cell divisions. A differentiated cell may derive from a multipotent cell which itself is derived from a multipotent cell, and so on. While each of these multipotent cells may be considered stem cells, the range of cell types each can give rise to may vary considerably. Some differentiated cells also have the capacity to give rise to cells of greater developmental potential. Such capacity may be natural or may be induced artificially upon treatment with various factors. By this definition, stem cells may also be progenitor cells, as well as the more immediate precursors to terminally differentiated cells.

"Proliferation" refers to an increase in cell number. "Proliferating" and "proliferation" refer to cells undergoing mitosis.

"Refractory" shall be taken to mean having no significant clinical benefit when used in the treatment of a diseases e.g. no significant improvement or amelioration of symptoms or subsequent relapse of disease.

The term rheumatoid arthritis is used herein in accordance with its normal meaning, namely an autoimmune disorder that results in inflammation, principally affecting the joints.

As used herein, the term "solution" includes a pharmaceutically acceptable carrier or diluent in which the MSCs used in the invention remain viable.

The term "substantially pure", with respect to MSC populations, refers to a population of cells in which at least about 75%, at least about 85%, at least about 90%, or at least about 95%, by number of the cells are MSCs. In other words, the term "substantially pure", with respect to MSC populations, refers to a population of cells that contains less than about 20%, less than about 10%, or less than about 5%, by number of lineage committed cells.

"Support" as used herein refers to any device or material that may serve as a foundation or matrix for the growth of adipose tissue-derived stromal stem cells.

"Therapeutic agent" or "therapeutic" refers to an agent capable of having a desired biological effect on a host. Chemotherapeutic and genotoxic agents are examples of therapeutic agents that are generally known to be chemical in origin, as opposed to biological, or cause a therapeutic effect by a particular mechanism of action, respectively. Examples of therapeutic agents of biological origin include growth factors, hormones, and cytokines. A variety of therapeutic agents are known in the art and may be identified by their effects. Certain therapeutic agents are capable of regulating cell proliferation and differentiation. Examples include chemotherapeutic nucleotides, drugs, hormones, non-specific (non-antibody) proteins, oligonucleotides (e.g., antisense oligonucleotides that bind to a target nucleic acid sequence (e.g., mRNA sequence)), peptides, and peptidomimetics.

The term "expressed" is used to describe the presence of a marker within a cell. In order to be considered as being expressed, a marker must be present at a detectable level. By "detectable level" is meant that the marker can be detected using one of the standard laboratory methodologies such as PCR, blotting or FACS analysis. The phenotypic surface marker characterization of a population of MSCs may be performed by any method known in the art. By way of example, but not limitation, this phenotypic characterization may be performed by individual cell staining. Such staining may be achieved through the use of antibodies. This may be direct staining, by using a labeled antibody or indirect staining, using a second labeled antibody against a primary antibody specific for the cell marker. Antibody binding may be detected by any method known in the art. Antibody binding may also be detected by flow cytometry, immunofluorescence microscopy or radiography.

Alternatively or additionally, a gene is considered to be expressed by a cell of the population of the invention if expression can be reasonably detected after 30 PCR cycles, which corresponds to an expression level in the cell of at least about 100 copies per cell. The terms "express" and "expression" have corresponding meanings. At an expression level below this threshold, a marker is considered not to be expressed. The comparison between the expression level of a marker in an adult stem cell of the invention, and the expression level of the same marker in another cell, such as for example an embryonic stem cell, may preferably be conducted by comparing the two cell types that have been isolated from the same species. Preferably this species is a mammal, and more preferably this species is human. Such comparison may conveniently be conducted using a reverse transcriptase polymerase chain reaction (RT-PCR) experiment.

### CELLS & COMPOSITIONS THEREOF

The invention provides a composition comprising a substantially pure population of expanded MSCs, wherein the MSCs are allogeneic adipose tissue derived stromal stem cells (ASCs), for use in a method of treating refractory rheumatoid arthritis in a human subject, wherein the composition is intravenously administered to the subject in repeated doses of about 1 million cells/kg at weekly intervals. The composition of the present invention may also include cell culture components, e.g., culture media including one or more of amino acids, metals and coenzyme factors. The composition may include small populations of other stromal cells. The composition may also include other non-cellular components which may support the growth and survival of the MSCs under particular circumstances, e.g. implantation, growth in continuous culture, or use as a biomaterial or composition.

The compositions of the invention comprise a population of cells in which at least about 75%, at least about 80%, at least about 85%, at least about 90%, at least about 95%, at least about 96%, at least about 97%, at least about 98%, or at least about 99%, of the cells are MSCs. In other words, at least about 75%, at least about 80%, at least about 85%, at least about 90%, at least about 95%, at least about 96%, at least about 97%, at least about 98%, or at least about 99%, of the cells in the composition are MSCs.

The compositions of the invention comprise at least about 75%, at least about 80%, at least about 85%, at least about 90%, at least about 95%, at least about 96%, at least about 97%, at least about 98%, or at least about 99%, MSCs, either calculated by number, or by weight or by volume of the composition.

The MSCs may express one or more (e.g. two or more, three or more, four or more, five or more, six or more, seven or more, eight or more, nine or more, or ten or more) of the markers CD9, CD10, CD13, CD29, CD44, CD49A, CD51, CD54, CD55, CD58, CD59, CD90 and CD105 at a significant level.

The MSCs may express one or more (e.g. two or more, three or more or four or more) of the markers CD9, CD44, CD54, CD90 and CD105.

The composition of the invention may comprise a population of cells comprising, consisting of, or consisting essentially of expanded adipose tissue derived stromal stem cells, wherein the majority of cells express CD59 and/or CD90, typically wherein expression of CD59 and/or CD90 within the population is greater than 50%, greater than 65%, or greater than 70%, or at least 95% e.g. 65-95%, or 70-90%. Typically, the composition of the invention comprises a population of cells comprising or consisting essentially of expanded adipose tissue derived stromal stem cells, wherein 70-90% of the cells express CD59 and CD90, e.g. wherein 75-90% of the cells express CD59 and 70-85% of the cells express CD90.

The MSC cell population used in the present invention may also be characterized in that the cells do not express a particular selection of markers at a detectable level. As defined herein, these markers are said be to be negative markers. In some embodiments, the stem cell population of the invention is considered not to express a marker if at least about 70% of the cells of the isolated adult stem cell population should not show detectable expression of the marker. In other embodiments, at least about 80%, at least about 90% or at least about 95% or at least about 97% or at least about 98% or at least about 99% or 100% of the cells of the stem cell population should not show any detectable expression of the marker. Again, lack of detectable expression may be proven through the use of an RT-PCR experiment or using FACS.

The composition of the invention may thus comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which do not express one or more (*e.g.* two or more, three or more, four or more, five or more, six or more, seven or more, eight or more, nine or more, or ten or more) of the markers CD34, CD11b, CD14, CD15, CD16, CD31, CD34, CD45, CD49f, CD102, CD104, CD106 and CD133 at a significant level.

The composition of the invention may thus comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which do not express one or more (*e.g*. two or more, three or more, four or more, five or more, six or more, seven or more, or eight or more) of the markers CD11b, CD1 1c, CD14, CD31, CD34, CD45, CD133 and HLAII at a significant level.

The composition of the invention may comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which do not express one or more (*e.g.* two or more, three or more, four or more, five or more, six or more, seven or more, eight or more, nine or more, or ten or more) of the markers CD34, STRO-1, CD11b, CD14, CD15, CD16, CD31, CD45, CD49f, CD102, CD104, CD106 and CD133 at a significant level. In one embodiment, the negative markers are CD34 and/or STRO-1. In another embodiment, the negative marker is CD34. In another embodiment, the negative marker is STRO-1. In another embodiment, the negative markers are CD34 and STRO-1.

The composition of the invention may comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which do not express one or more (*e.g.* two or more, three or more, four or more, five or more, six or more, seven or more, or eight or more) of the markers CD11b, CD11c, CD14, CD31, CD34, STRO-1, CD45, CD133 and HLAII at a significant level. In one embodiment, the negative markers are CD34 and/or STRO-1. In another embodiment, the negative marker is CD34. In another embodiment, the negative marker is STRO-1. In another embodiment, the negative markers are CD34 and STRO-1.

In one embodiment, less than 50%, less than 35%, less than 30%, or less than 5% (by number of cells, or by weight or volume of the composition) of cells of the composition express CD34 at a significant level, e.g. 35-5%, or 20-10% of cells express CD34 at a significant level. Typically, less than 5% of cells of the composition express CD34 at a significant level.

In one embodiment, less than 50%, less than 35%, less than 30%, or less than 5% (by number of cells, or by weight or volume of the composition) of cells of the composition express STRO-1 at a significant level, e.g. 35-5%, or 20-10% of cells express STRO-1 at a significant level. Typically, less than 5% of cells of the composition express STRO-1 at a significant level.

Typically, less than 50%, less than 35%, less than 30%, or less than 5% (by number of cells, or by weight or volume of the composition) of cells of the composition express CD34 and STRO-1 at significant levels, e.g. 35-5%, or 20-10% of cells express CD34 and STRO-1 at significant levels. Typically, less than 5% of cells of the composition express CD34 and STRO-1 at significant levels.

The composition of the invention may comprise a population of cells comprising, consisting of, or consisting essentially of expanded adipose tissue derived stromal stem cells, wherein the majority of cells do not express CD34, typically wherein expression of CD34 within the population is less than 50%, less than 35%, or less than 30%, or less than 5% e.g. 35-5%, or 20-10%. Typically, the composition of the invention comprises a population of cells comprising or consisting essentially of expanded adipose tissue derived stromal stem cells, wherein 70-90% of the cells do not express CD34, e.g. wherein 75-90% of the cells do not express CD34.

The composition of the invention may comprise a population of cells comprising, consisting of, or consisting essentially of expanded adipose tissue derived stromal stem cells, wherein the majority of cells do not express STRO-1, typically wherein expression of STRO-1 within the population is less than 50%, less than 35%, or less than 30%, or less than 5% e.g. 35-5%, or 20-10%. Typically, the composition of the invention comprises a population of cells comprising or consisting essentially of expanded adipose tissue derived stromal stem cells, wherein 70-90% of the cells do not express STRO-1, e.g. wherein 75-90% of the cells do not express STRO-1.

Typically, the composition of the invention comprises a population of cells comprising, consisting of, or consisting essentially of expanded adipose tissue derived stromal stem cells, wherein the majority of cells do not express CD34 and STRO-1, typically wherein expression of CD34 and STRO-1 within the population is less than 50%, less than 35%, or less than 30%, or less than 5% e.g. 35-5%, or 20-10%. Typically, the composition of the invention comprises a population of cells comprising or consisting essentially of expanded adipose tissue derived stromal stem cells, wherein 70-90% of the cells do not express CD34 and STRO-1, e.g. wherein 75-90% of the cells do not express CD34 and STRO-1.

The composition of the invention may comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which express one or more (*e.g.* two or more, or all three) of the markers c-Kit, vimentin and CD90 at a significant level. In one preferred embodiment, a composition of the invention comprises 95% or more cells with express c-Kit, vimentin and CD90 at a significant level.

The composition of the invention may comprise at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% (by number of cells, or by weight or volume of the composition), MSCs which do not express one or more (*e.g.* two or more, three or more, four or more, five or more, or all six) of the markers CD34, Factor VIII, alpha-actin, desmin, S-100 and keratin at a significant level.

The composition of the invention comprises an expanded cell population.

Typically, said cell populations are expanded until expression of the marker CD34 is reduced compared to freshly isolated, non-expanded cells. For example the cell population is expanded until expression of the marker CD34 is reduced to a level of less than 50%, less than 35%, less than 30%, or less than 5%, e.g. 35-5%, or 20-10%, of cells in the population. Typically, the cell population is expanded until expression of the marker CD34 is reduced to a level of less 5% of cells in the population.

Typically, said cell populations are expanded until expression of the marker STRO-1 is reduced compared to freshly isolated, non-expanded cells. For example the cell population is expanded until expression of the marker STRO-1 is reduced to a level of less than 50%, less than 35%, less than 30%, or less than 5%, e.g. 35-5%, or 20-10%, of cells in the population. Typically, the cell population is expanded until expression of the marker STRO-1 is reduced to a level of less 5% of cells in the population.

In one embodiment, the cell populations are expanded until expression of the markers CD34 and STRO-1 is reduced compared to freshly isolated, non-expanded cells. For example the cell population is expanded until expression of the markers CD34 and STRO-1 is reduced to a level of less than 50%, less than 35%, less than 30%, or less than 5%, e.g. 35-5%, or 20-10%, of cells in the population. Typically, the cell population is expanded until expression of the markers CD34 and STRO-1 is reduced to a level of less 5% of cells in the population.

Expanded MSC populations (e.g. those expressing 5% or less CD34 and/or STRO-1) are advantageous as they present a lower multipotency than freshly isolated cells, i.e. they may have a lower, reduced or no capacity to differentiate into other cell phenotypes as compared to naturally-occurring non-expanded MSCs. The concentration of the MSCs in the composition of the invention may be at least about 1 x 10⁴ cells/mL, at least about 1 x 10⁵ cells/mL, at least about 1 x 10⁶ cells/mL, at least about 10 x 10⁶ cells/mL, or at least about 40 x 10⁶ cells/mL.

In some embodiments, at least about 40% (*e.g.* at least about 50%, at least about 60%, at least about 70%, at least about 80%, at least about 85%, at least about 90%, at least about 95% at least about 96%, at least about 97%, at least about 98%, or at least about 99%) of the MSCs in a composition of the invention are pre-stimulated in order to enhance one or more of their proliferation capacity, migration capacity, survival capacity, therapeutic effect and inmunoregulatory properties. In some embodiments, pre-stimulation may be achieved by contacting the MSCs with a cytokine. In some embodiments of the invention, pre-stimulation may be achieved by contacting the MSCs with IFN-γ.

In certain embodiments of the invention, the MSCs may be pre-stimulated using a concentration of IFN-γ between 0.1 and 100 ng/ml. In further embodiments, the MSCs may be pre-stimulated using a concentration of IFN-γ between 0.5 and 85 ng/ml, between 1 and 70 ng/ml, between 1.5 and 50 ng/ml, between 2.5 and 40 ng/ml, or between 3 and 30 ng/ml. Pre-stimulation may occur over a stimulation time longer than about 12 hours. Pre-stimulation may occur over a stimulation time longer than about 13 hours, longer than about 18 hours, longer than about 24 hours, longer than about 48 hours, or longer than about 72 hours.

In some embodiments of the invention, the MSCs may be characterized in that they:
a) do not express markers specific for antigen-presenting cells (APC),
b) do not express indoleamine 2,3-dioxygenase (IDO) constitutively, and
c) express IDO upon stimulation with interferon-gamma (IFN-γ).

MSCs useful in the context of the present invention have the ability to inhibit T cell activation. T cell activation can be measured by cytokine secretion and T cell proliferation. For example, MSCs of the invention preferably fail to elicit proliferation or secretion of IFNγ when co-cultured with unmatched PBMCs. MSCs of the invention may also inhibit secretion of IFNγ and the proliferation of PBMCs stimulation with the superantigen SEB.

### RHEUMATOID ARTHRITIS TREATMENTS

As is detailed in the examples, treatment with MSCs (in this case, hASCs) was found to ameliorate the symptoms of rheumatoid arthritis. It is demonstrated that treatment with hASC in a dose range of up to about 4 x 10⁶ cells/kg ameliorated symptoms of rheumatoid arthritis. Surprisingly it was found that lower doses of ASC provided greater clinical benefit. The invention provides a composition comprising a substantially pure population of expanded mesenchymal stem cells (MSCs), wherein the MSCs are allogeneic adipose tissue derived stromal stem cells (ASCs), for use in a method of treating refractory rheumatoid arthritis in a human subject, wherein the composition is intravenously administered to the subject in repeated doses of about 1 million cells/kg at weekly intervals optionally on days 1, 8 and 15.

As shown in the Examples the hASCs treatment of the invention was demonstrated as ameliorating the symptoms of rheumatoid arthritis patients who were refractory to standard treatment. Accordingly in a preferred embodiment of the invention the hASCs are used in treating rheumatoid arthritis in patients refractory to at least one, two or three therapies for treatment of rheumatoid arthritis. Said therapies may include one or more of disease-modifying antirheumatic drugs (DMARDs), non-steroidal anti-inflammatories (NSAIDs), biological medicinal products and corticosteroids. Disease-modifying antirheumatic drugs (DMARDs), include but are not limited to auranofin, choroquine, cyclosporine, cyclophosphamide, gold preparations, hydroxychloroquine sulphate, leflunomide, methotrexate, penicillamine, sodium aurothiomalate, sulfasalazine. In one embodiment it is particularly preferred that the patients are treatment refractory to at least methotrexate. In one embodiment it is particularly preferred that the patients are treatment refractory to at least leflunomide. In a further embodiment it is preferred that the patients are treatment refractory to at least both of leflunomide and methotrexate. Biological medicinal products, include but are not limited to selective costimulation modulators, Tnf-a inhibitors, IL-1 inhibitors, IL-6 inhibitors & B-cell targeted therapies. Exemplary biological medicinal products are listed in Table 3. Typically, the patient is refractory to at least one biological treatment, for example any one of the biologicals listed in Table 3, for example a TNF-α inhibitor. Accordingly, in one aspect of the invention the patient is refractory to at least one biological treatment which is a TNF-α inhibitor, typically Adalimumab (Humira), Certolizumab (Cimzia), Etanercept (Enbrel), Golimumab (Simponi), or Infliximab (Remicade). Typically, the patient is refractory to at least two biological treatments, for example any two of the biologicals listed in Table 3, optionally at least one of which may be a TNF-α inhibitor. In one embodiment the patients are treatment refractory to at least methotrexate and a TNF-α inhibitor; wherein it is particularly preferred that said TNF-α inhibitor is selected from the group comprising Adalimumab, Etanercept & Infliximab.
In a further embodiment the patients are treatment refractory to at least leflunomide and a TNF-α inhibitor; wherein it is particularly preferred that said TNF-α inhibitor is selected from the group comprising Adalimumab, Etanercept & Infliximab. In a further embodiment the patients are treatment refractory to at least leflunomide, methotrexate and a TNF-α inhibitor; wherein it is particularly preferred that said TNF-α inhibitor is selected from the group comprising Adalimumab, Etanercept & Infliximab.

In one embodiment, there is disclosed herein a composition comprising a population of cells comprising, consisting of, or consisting essentially of expanded adipose tissue derived stromal stem cells, wherein the majority of cells express CD59 and/or CD90, typically wherein expression of CD59 and/or CD90 within the population is greater than 50%, greater than 65%, or greater than 70%, e.g. 65-95%, or 70-90%, for treating rheumatoid arthritis in a subject, wherein the subject is refractory to at least one biological TNF-α inhibitor, wherein the composition comprises between about 0.5 x 10⁶ cells/kg to about 2 x 10⁶ cells/kg, or more preferably about 1 x 10⁶ cells/kg to about 2 x 10⁶ cells/kg, or about 2 x 10⁶ cells/kg or lower, most preferably about 1 x 10⁶ cells/kg, and wherein each dose is administered intravenously at days 1, 8 and 15.

In a further embodiment, there is disclosed herein a composition comprising a population of cells comprising, consisting of, or consisting essentially of expanded adipose tissue derived stromal stem cells, wherein the majority of cells do not express CD34, typically wherein expression of CD34 within the population is less than 50%, less than 35%, or less than 30%, e.g. 35-5%, or 20-10%, or most preferably less than 5% of cells, for treating rheumatoid arthritis in a subject, wherein the subject is refractory to at least one biological TNF-α inhibitor, wherein the composition comprises between about 0.5 x 10⁶ cells/kg to about 2 x 10⁶ cells/kg, or more preferably about 1 x 10⁶ cells/kg to about 2 x 10⁶ cells/kg, or about 2 x 10⁶ cells/kg or lower, most preferably about 1 x 10⁶ cells/kg, and wherein each dose is administered intravenously at days 1, 8 and 15.

In a further embodiment, there is disclosed herein a composition comprising a population of cells comprising, consisting of, or consisting essentially of expanded adipose tissue derived stromal stem cells, wherein the majority of cells do not express STRO-1, typically wherein expression of STRO-1 within the population is less than 50%, less than 35%, or less than 30%, e.g. 35-5%, or 20-10%, or most preferably less than 5% of cells, for treating rheumatoid arthritis in a subject, wherein the subject is refractory to at least one biological TNF-α inhibitor, wherein the composition comprises between about 0.5 x 10⁶ cells/kg to about 2 x 10⁶ cells/kg, or more preferably about 1 x 10⁶ cells/kg to about 2 x 10⁶ cells/kg, or about 2 x 10⁶ cells/kg or lower, most preferably about 1 x 10⁶ cells/kg, and wherein each dose is administered intravenously at days 1, 8 and 15.

In a further embodiment, there is disclosed herein a composition comprising a population of cells comprising, consisting of, or consisting essentially of expanded adipose tissue derived stromal stem cells, wherein the majority of cells do not express CD34 and STRO-1, typically wherein expression of CD34 and STRO-1 within the population is less than 50%, less than 35%, or less than 30%, e.g. 35-5%, or 20-10%, or most preferably less than 5% of cells, for treating rheumatoid arthritis in a subject, wherein the subject is refractory to at least one biological TNF-α inhibitor, wherein the composition comprises between about 0.5 x 10⁶ cells/kg to about 2 x 10⁶ cells/kg, or more preferably about 1 x 10⁶ cells/kg to about 2 x 10⁶ cells/kg, or about 2 x 10⁶ cells/kg or lower, most preferably about 1 x 10⁶ cells/kg, and wherein each dose is administered intravenously at days 1, 8 and 15.

In a further embodiment, there is disclosed herein a composition comprising a population of cells comprising, consisting of, or consisting essentially of expanded adipose tissue derived stromal stem cells, wherein expression of CD34, STRO-1, or CD34 and STRO-1 within the population is less than 5% of cells, for treating rheumatoid arthritis in a subject, wherein the subject is refractory to at least one biological TNF-α inhibitor, wherein the composition comprises about 1 x 10⁶ cells/kg, and wherein each dose is administered intravenously at days 1, 8 and 15.

The composition of the invention preferably comprises expanded MSC. Accordingly said composition may comprise the progeny of MSCs. Such progeny may include subsequent generations of MSCs, preferably undifferentiated progeny. It will be understood that progeny cells may be obtained after any number of population doublings from the parental population. Where the expansion is carried out by passaging the MSC progeny cells may be obtained after any number of passages from the parental population. However, in certain embodiments, the progeny cells may be obtained after about 2, about 3, about 4, about 5, about 6, about 7, about 8, about 9, or about 10 passages from the parental population.

A composition of the invention may be provided under sterile conditions, and may be free of viruses, bacteria and other pathogens. A composition of the invention may be provided as a pyrogen-free preparation.

The composition of the invention is administered intravenously.

The MSCs used in the invention are allogeneic with respect to the subject to be treated. Previous studies have shown that allogeneic bone marrow-derived stromal stem cells and adipose tissue-derived stromal cells do not provoke a lymphocyte immune response when brought into contact with allogeneic lymphocytes *in vitro.* Consequently, allogenic adipose tissue-derived stromal stem cells derived from a donor may theoretically be used for the treatment of any patient, irrespective of MHC incompatibility. In embodiments wherein allogeneic stem cells are used, supportive treatment may be required. For example, immunosuppressants may be administered before, during and/or after treatment to prevent Graft-Versus-Host-Disease (GVHD), according to known methods. Prior to administration, the cells may also be modified to suppress an immune reaction from the subject to the cells or vice-versa, according to methods known in the art.

In one embodiment, the composition of the invention may be administered by injection or implantation of the composition at one or more target sites in the subject to be treated. In a further embodiment, the composition of the invention may be inserted into a delivery device which facilitates introduction of the composition into the subject by injection or implantation. In one embodiment the delivery device may comprise a catheter. In a further embodiment, the delivery device may comprise a syringe.

The compositions of the invention will generally comprise a pharmaceutically acceptable carrier and/or a diluent. Examples of such carriers and diluents are well known in the art, and may include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

A composition of the invention may be sterile and fluid to the extent that easy syringability exists. In addition, the composition may be stable under the conditions of manufacture and storage and preserved against the contaminating action of microorganisms such as bacteria and fungi through the use of, for example, parabens, chlorobutanol, phenol, ascorbic acid and thimerosal.

In one embodiment, the pharmaceutical composition of the invention may contain one or more (or two or more, or three or more, e.g. 1, 2, 3, 4 or 5) further therapeutic agents, such as a therapeutic agent selected from the following: an analgesic, such as a nonsteroidal anti-inflammatory drug, an opiate agonist or a salicylate; an anti-infective agent, such as an antihelmintic, an antianaerobic, an antibiotic, an aminoglycoside antibiotic, an antifungal antibiotic, a cephalosporin antibiotic, a macrolide antibiotic, a β-lactam antibiotic, a penicillin antibiotic, a quinolone antibiotic, a sulfonamide antibiotic, a tetracycline antibiotic, an antimycobacterial, an antituberculosis antimycobacterial, an antiprotozoal, an antimalarial antiprotozoal, an antiviral agent, an anti-retroviral agent, a scabicide, an anti-inflammatory agent, a corticosteroid anti-inflammatory agent, an antipruritics/local anesthetic, a topical anti-infective, an antifungal topical anti-infective, an antiviral topical anti-infective; an electrolytic and renal agent, such as an acidifying agent, an alkalinizing agent, a diuretic, a carbonic anhydrase inhibitor diuretic, a loop diuretic, an osmotic diuretic, a potassium-sparing diuretic, a thiazide diuretic, an electrolyte replacement, and an uricosuric agent; an enzyme, such as a pancreatic enzyme and a thrombolytic enzyme; a gastrointestinal agent, such as an antidiarrheal, an antiemetic, a gastrointestinal anti-inflammatory agent, a salicylate gastrointestinal anti-inflammatory agent, an antacid anti-ulcer agent, a gastric acid-pump inhibitor anti-ulcer agent, a gastric mucosal anti-ulcer agent, a H2-blocker anti-ulcer agent, a cholelitholytic agent, a digestant, an emetic, a laxative and stool softener, and a prokinetic agent; a general anesthetic, such as an inhalation anesthetic, a halogenated inhalation anesthetic, an intravenous anesthetic, a barbiturate intravenous anesthetic, a benzodiazepine intravenous anesthetic, and an opiate agonist intravenous anesthetic; a hormone or hormone modifier, such as an abortifacient, an adrenal agent, a corticosteroid adrenal agent, an androgen, an anti-androgen, an immunobiologic agent, such as an immunoglobulin, an immunosuppressive, a toxoid, and a vaccine; a local anesthetic, such as an amide local anesthetic and an ester local anesthetic; a musculoskeletal agent, such as an anti-gout anti-inflammatory agent, a corticosteroid anti-inflammatory agent, a gold compound anti-inflammatory agent, an immunosuppressive anti-inflammatory agent, a non-steroidal anti-inflammatory drug (NSAID), a salicylate anti-inflammatory agent, a mineral; and a vitamins, such as vitamin A, vitamin B, vitamin C, vitamin D, vitamin E, and vitamin K.

In another embodiment, the further therapeutic agent may be a growth factor or other molecule that affects cell proliferation or activation. In a further embodiment that growth factor may induce final differentiation. In another embodiment, the growth factor may be a variant or fragment of a naturally-occurring growth factor. Methods of producing such variants are well known in the art, and may include, for example, making conservative amino acid changes, or by mutagenesis and assaying the resulting variant for the required functionality.

In one embodiment, MSCs may be administered to a subject in conjunction with one or more (or two or more, or three or more, e.g. 1, 2, 3, 4 or 5) further therapeutic agents. In some embodiments, the MSCs and the one or more further therapeutic agents may be administered to the subject simultaneously. In other embodiments, the MSCs and the one or more further therapeutic agents may be administered to the subject sequentially. The one or more further therapeutic agents may be administered before or after administration of the MSCs.

Preferably the one or more therapeutic agents are selected from the group consisting of disease-modifying antirheumatic drugs (DMARDs), non-steroidal anti-inflammatories (NSAIDs), biological medicinal products and corticosteroids. Disease-modifying antirheumatic drugs (DMARDs), include but are not limited to auranofin, choroquine, cyclosporine, cyclophosphamide, gold preparations, hydroxychloroquine sulphate, leflunomide, methotrexate, penicillamine, sodium aurothiomalate, sulfasalazine. Biological medicinal products, include but are not limited to selective costimulation modulators, Tnf-a inhibitors, IL-1 inhibitors, IL-6 inhibitors & B-cell targeted therapies.

The compositions of the invention are administered in repeated doses at a dosage of about 1 million cells/kg at weekly intervals, preferably on days 1, 8 and 15 of treatment. Surprisingly it was found that lower doses of hASC provided greater clinical benefit.

In one embodiment, a dose of 1 million mesenchymal stem cells/kg is administered to a human patient at days 1, 8 and 15. The patient has refractory rheumatoid arthritis, which may optionally be aggravated. Typically, the patient is refractory to at least two biological treatments, for example any two of the biologicals listed in Table 3. The MSCs are expanded Adipose-derived Mesenchymal Stem Cells (eASCs), which are administered intravenously.

### CELL PREPARATION METHODS

In one embodiment the disclosure provides a method of preparing a composition, which comprises: (a) collecting MSCs from a subject; (b) obtaining a cell suspension by enzymatic treatment; (c) sedimenting the cell suspension and re-suspending the cells in a culture medium; (d) culturing the cells for at least about 10 days; and (g) expanding the cells for at least two culture passages.

MSCs for use in the invention are isolated from adipose tissue of a human who is different to the human subject into which the composition of the invention is to be introduced. In one aspect of the disclosure, adipose-derived MSCs can be obtained essentially as described by Zuk et al., 2001. According to this methodology, lipoaspirates are obtained from adipose tissue and the cells derived therefrom. In the course of this methodology, the cells may preferably be washed to remove contaminating debris and red blood cells, preferably with PBS. The cells are preferably digested with collagenase (e.g. at 37°C for 30 minutes, 0.075% collagenase; Type I, Invitrogen, Carlsbad, CA) in PBS. To eliminate remaining red blood cells, the digested sample can be washed (e.g. with 10% fetal bovine serum), treated with 160 mmol/L C1NH4, and finally suspended in DMEM complete medium (DMEM containing 10% FBS, 2 mmol/L glutamine and 1% penicillin/streptomycin). The cells can be filtered through a 40-µm nylon mesh. Cells isolated in this way can be seeded (preferably 2-3x104 cells/cm2) onto tissue culture flasks and expanded at 37°C and 5% CO2, changing the culture medium every 3-4 days. Cells are preferably passed to a new culture flask (1,000 cells/cm2) when cultures reach 90% of confluence.

In certain embodiments, the cells may be cultured for at least about 15 days, at least about 20 days, at least about 25 days, or at least about 30 days. The expansion of cells in culture may improve the homogeneity of the cell phenotype in the cell population.

In certain embodiments, the cells are expanded in culture for at least three culture passages or "passaged at least three times". In other embodiments, the cells are passaged at least four times, at least five times, at least six times, at least seven times, at least eight times, at least nine times, or at least ten times. The cells may be expanded in culture indefinitely provided that the homogeneity of the cell phenotype is improved and differential capacity is maintained. Alternatively, the differentiation capacity of the expanded cells may be reduced compared to freshly isolated or naturally occurring, non-expanded cells.

Cells may be cultured by any technique known in the art for the culturing of stem cells. A discussion of various culture techniques, as well as their scale-up, may be found in Freshney, R.I., Culture of Animal Cells: A Manual of Basic Technique, 4th Edition, Wiley-Liss 2000. Cells may be expanded using culture flasks or bioreactors suitable for large-scale expansion. Bioreactors suitable for the large-scale expansion of mesenchymal stem cells are commercially available and may include both 2D and 3D expansion bioreactors. Examples of such bioreactors include, but are not limited to, a plug flow bioreactor, a perfusion bioreactor, a continuous stirred tank bioreactor, a stationary-bed bioreactor.

In certain embodiments, the cells are cultured by monolayer culture. Any medium capable of supporting MSCs in tissue culture may be used. Media formulations that will support the growth of MSCs include, but are not limited to, Dulbecco's Modified Eagle's Medium (DMEM), alpha modified Minimal Essential Medium (αMEM), and Roswell Park Memorial Institute Media 1640 (RPMI Media 1640). Typically, 0 to 20% Fetal Bovine Serum (FBS) will be added to the above media in order to support the growth of stromal cells. However, a defined medium could be used if the necessary growth factors, cytokines, and hormones in FBS for stromal cells and chondrocytes are identified and provided at appropriate concentrations in the growth medium. Media useful in the methods of the invention may contain one or more compounds of interest, including, but not limited to antibiotics, mitogenic or differentiating compounds for stromal cells. The cells of the invention may be grown at temperatures between 31°C to 37°C in a humidified incubator. The carbon dioxide content may be maintained between 2% to 10% and the oxygen content may be maintained at between 1% and 22%. Cells may remain in this environment for periods of up to about 4 weeks.

Antibiotics which can be added to the medium include, but are not limited to penicillin and streptomycin. The concentration of penicillin in the chemically defined culture medium may be about 10 to about 200 units per ml. The concentration of streptomycin in the chemically defined culture medium may be about 10 to about 200 ug/ml.

In one embodiment, the MSCs of the invention may be stably or transiently transfected or transduced with a nucleic acid of interest using a plasmid, viral or alternative vector strategy. Nucleic acids of interest include, but are not limited to, those encoding gene products which enhance the production of extracellular matrix components found in the tissue type to be repaired, e.g. intestinal wall or vaginal wall.

The transduction of viral vectors carrying regulatory genes into the stromal stem cells can be performed with viral vectors, including but not limited to adenovirus, retrovirus or adeno-associated virus purified (e.g. by cesium chloride banding) at a multiplicity of infection (viral units:cell) of between about 10:1 to 2000:1. Cells may be exposed to the virus in serum free or serum-containing medium in the absence or presence of a cationic detergent such as polyethyleneimine or Lipofectamine™ for a period of about 1 hour to about 24 hours (Byk T. et al. (1998) Human Gene Therapy 9:2493-2502; Sommer B. et al. (1999) Calcif. Tissue Int. 64:45-49).

Other suitable methods for transferring vectors or plasmids into stem cells include lipid/DNA complexes, such as those described in U.S. Pat. Nos. 5,578,475; 5,627,175; 5,705,308; 5,744,335; 5,976,567; 6,020,202; and 6,051,429. Suitable reagents include lipofectamine, a 3:1 (w/w) liposome formulation of the poly-cationic lipid 2,3-dioleyloxy-N-[2(sperminecarbox-amido)ethyl]-N,N-dimethyl-1-propanaminium trifluoroacetate (DOSPA) (Chemical Abstracts Registry name: N-[2-(2,5-bis[(3-aminopropyl)amino]-1--oxpentyl}amino) ethyl]-N,N-dimethyl-2,3-bis(9-octadecenyloxy)-1-propanaminium trifluoroacetate), and the neutral lipid dioleoyl phosphatidylethanolamine (DOPE) in membrane filtered water. Exemplary is the formulation Lipofectamine 2000TM (available from Gibco/Life Technologies # 11668019). Other reagents include: FuGENETM 6 Transfection Reagent (a blend of lipids in non-liposomal form and other compounds in 80% ethanol, obtainable from Roche Diagnostics Corp. # 1814443); and LipoTAXITM transfection reagent (a lipid formulation from Invitrogen Corp., #204110). Transfection of stem cells can be performed by electroporation, e.g., as described in M.L. Roach and J.D. McNeish (2002) Methods in Mol. Biol. 185:1. Suitable viral vector systems for producing stem cells with stable genetic alterations may be based on adenoviruses and retroviruses, and may be prepared using commercially available virus components.

The transfection of plasmid vectors carrying regulatory genes into the MSCs can be achieved in monolayer cultures by the use of calcium phosphate DNA precipitation or cationic detergent methods (Lipofectamine™, DOTAP) or in three dimensional cultures by the incorporation of the plasmid DNA vectors directly into the biocompatible polymer (Bonadio J. et al. (1999) Nat. Med. 5:753-759).

For the tracking and detection of functional proteins encoded by these genes, the viral or plasmid DNA vectors may contain a readily detectable marker gene, such as the green fluorescent protein or beta-galactosidase enzyme, both of which can be tracked by histochemical means.

Subsequent to expansion it is preferred that the cell compositions are assayed to determine their phenotype, which can be carried out by using conventional means. Cell-surface markers can be identified by any suitable conventional technique, usually based on a positive/negative selection; for example, monoclonal antibodies against cell-surface markers, whose presence/absence in the cells is to be confirmed, can be used; although other techniques can also be used. Thus, in a particular embodiment, monoclonal antibodies against one, two, three, four, five, six, seven of or all of Factor VIII, alpha-actin, desmin, S-100, keratin CD11b, CD11c, CD14, CD45, HLAII, CD31, CD34, STRO-1 and CD133 are used in order to confirm the absence of said markers in the selected cells; and monoclonal antibodies against one, two, three, four, of or all of CD10, CD13, CD29, CD49A, CD51, CD55, CD58, CD59, c-Kit, vimentin, CD9, CD44, CD54, CD90 and CD105 are used in order to confirm the presence thereof or detectable expression levels of, at least one of and preferably all of, said markers.

In a further embodiment, monoclonal antibodies against CD34 is used in order to confirm the absence of said marker in the selected cells. In a further embodiment, monoclonal antibodies against STRO-1 is used in order to confirm the absence of said marker in the selected cells. In a further embodiment monoclonal antibodies against CD34 and STRO-1 are used in order to confirm the presence thereof or detectable expression levels of both markers. In a further embodiment monoclonal antibodies against CD59 and/or CD90 are used in order to confirm the presence thereof or detectable expression levels of, at least one of and preferably both of, said markers.

Said monoclonal antibodies are known, commercially available or can be obtained by a skilled person in the art by conventional methods.

IPN-γ-inducible IDO activity in the selected cells can be determined by any suitable conventional assay. For example, the selected cells can be stimulated with IFN-γ and assayed for IDO expression; then conventional Western-blot analysis for IDO protein expression can be performed and IDO enzyme activity following IFN-γ stimulation of the selected cells can be measured by tryptophan-to-kynurenine conversion with for example via High Performance Liquid Chromatography (HPLC) analysis and photometric determination of kynurenine concentration in the supernatant as the readout. Since the cells of the invention express IDO under certain conditions, any suitable technique which allows the detection of IDO activity following IFN-γ stimulation may be used for selecting the cells of the invention. A suitable assay for determining IFN-γ-inducible IDO activity in the selected cells is disclosed in WO2007039150. The amount of IDO produced depends on the number of cells per square centimetre, which is preferably at a level of 5000cells/cm² or more, but not limited to this concentration and the concentration of IFN-γ, which ideally is 3ng/ml or more, but not limited to this concentration. The activity of IDO produced under the described conditions will result in a detectable production of kynurenine in the µM range after 24hours or more.

The invention will now be further illustrated by the following examples. These examples are provided by way of illustration only, and are not intended to be limiting.

### EXAMPLES

Aim: Expanded adipose-derived stromal cells (eASCs) are shown to have immune-modulatory effects both in vitro and in animal models of arthritis. The aim of the present study was to determine the safety, feasibility and tolerance of intravenous (IV) administration of allogeneic eASCs in RA patients.

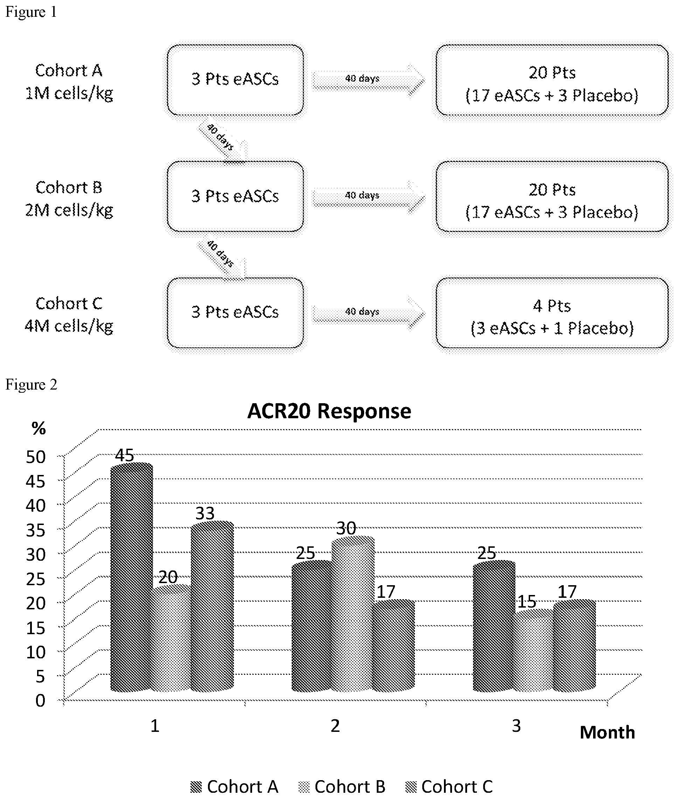

Methods: A 24-week, single blind dose-escalating study in RA patients under treatment with at least one non-biological DMARD and who previously failed treatment with at least two biologicals was conducted in 23 centers (Figure 1).

### eASC dosage

Fifty-three patients with moderate to high disease activity (Table 1 and Table la; DAS28>3.2) were assigned to receive 1x10⁶ eASCs/kg body weight (cohort A: 20 patients), 2x10⁶ eASCs/kg body weight (cohort B: 20 patients), 4x10⁶ eASCs/kg body weight (cohort C: 6 patients) or placebo (Ringer's lactate solution: 7 patients). All patients received 3 IV eASC infusions at day 1, 8 and 15. Tolerability and treatment emergent adverse events such as Dose Limiting Toxicities (DLTs), serious adverse events (SAEs) and non-serious adverse events (AEs) were primary endpoints. ACR20/50/70, DAS 28, and SF-36 were secondary endpoints.

The study group was considered a difficult to treat population of extremely refractory patients as the majority had been treated with all commonly used therapies (see Table 1b and Table 2). Table 3 shows the percentage of patients treated with biological medicinal products; 45% of patients received 3 or more biologics (mean 2.92, standard deviation 1.44). Table 4 shows the percentage of patients previously treated with DMARDS, 74% of patients received 3 or more DMARDs (mean 3.38, standard deviation 1.51).

### Isolation and expansion of eASCs

The allogeneic eASCs medicinal product consists of a cellular suspension of living adult stem cells of mesenchymal origin extracted from the subdermal adipose tissue of healthy donors. Subdermal adipose tissue was liposuctioned from the healthy donor and transported to the manufacturing facility. The donation, procurement, and testing were carried out according to the requirements of Directive 2004/23/EC and therefore under Directives 2006/17/EC and 2006/83/EC. ASCs were isolated by digesting the adipose tissue with type I collagenase, followed by centrifugation. The cell pellet obtained was resuspended and lysed in erythrocyte lysis solution and centrifuged. The stromal vascular fraction, resulting from the cell pellet, was placed in cell culture containers in culture medium and antibiotics, and incubated at 37 °C and 5 % CO2 and in a humidified atmosphere. At 24-48 h post-plating, the culture medium was removed to eliminate the non-attached cell fraction. ASCs adhered to the plastic culture plates that were expanded under in vitro conditions. Every 3-4 days, the culture medium was changed after reaching 90-95 % confluence and the cells were detached with trypsin/EDTA, collected, centrifuged, and expanded without antibiotics to the required duplication. They were then harvested and cryopreserved until use. Before the appointed administration date, sufficient cryopreserved vials were thawed to provide the required dose for administration. ASCs were recovered from their cryopreserved state by plating and culturing (to confirm viability). On the day when the vials were filled and packaged, the cultures were washed with phosphate buffer solution, and trypsin/EDTA. The ASCs were immediately resuspended in the selected excipients (Dulbecco modification Eagle medium and human albumin serum) to formulate the drug product.

Cell characterization The eASCs were characterized in terms of identity (phenotypic profile), purity, potency, morphology, viability, and cell growth kinetics according to the Guideline on Cell- Based Medicinal Products (EMEA/CHMP/410869/2006) and the Reflection Paper on Stem Cells (EMA/CAT/ 571134/2009).

Results: Patient and disease characteristics were comparable for all three dose groups, with DAS28 (CRP) disease activity scores between 3.2 and 7.9. Time since onset of the disease was between 3 and 69 years (median 13 years). Repeated IV infusion of eASCs did not show any major safety signals and no dose-limiting safety signal was identified.

In secondary endpoints, maximum clinical benefit in the ITT (intention to treat) population was observed in cohort A.

The highest proportion of ACR20 patients in the ITT population was observed one month after the first treatment dose, with 45%, 20%, 33% and 28% of the patients reaching ACR20 in the cohorts A, B, C and placebo; respectively (p=0.3979). Per treatment group, the highest rates were at one month after the first treatment dose for cohorts A, C and placebo, and at two months after the first treatment dose for cohort B (30%) (Figure 2).

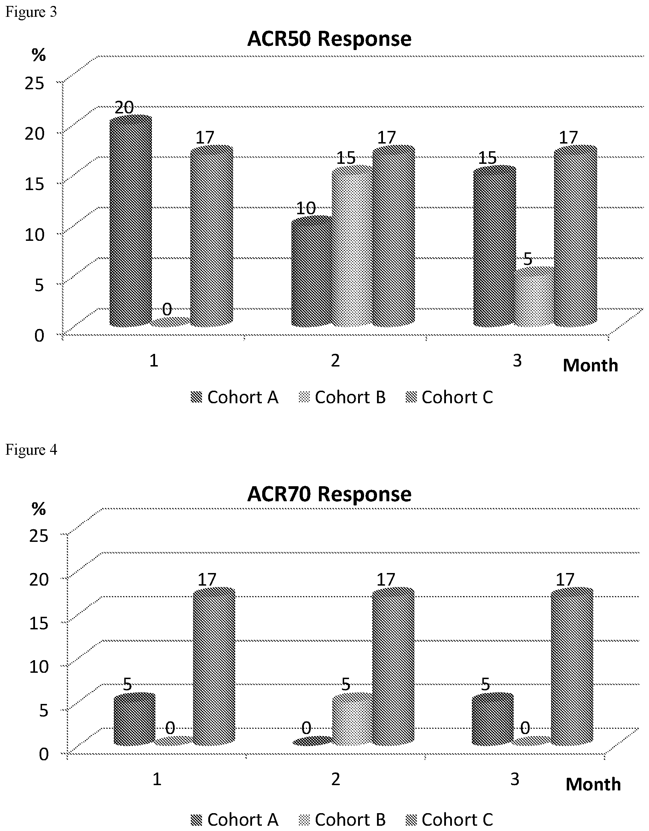

A similar trend was observed for both ACR50 (Figure 3) and ACR70 (Figure 4). However, in the cohort B no ACR50/70 responses were observed before V5. Of note, with the exception of one patient with an ACR50 response at V4, there were not ACR50/70 responses during the trial in the placebo group. Overall, a higher proportion of patients treated with eASCs, compared with those patients treated with placebo, achieved ACR20, ACR50 and ACR70 during the trial.

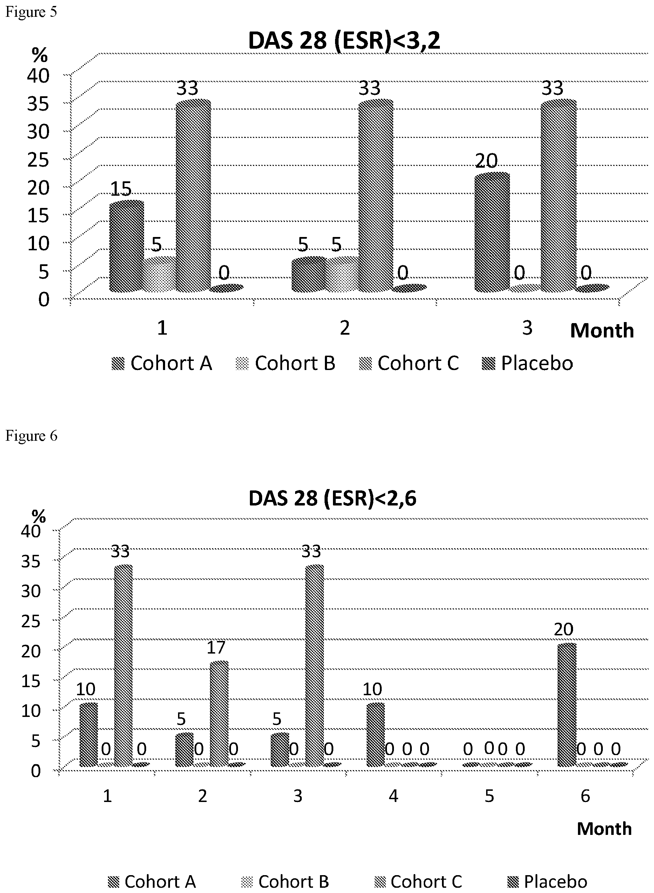

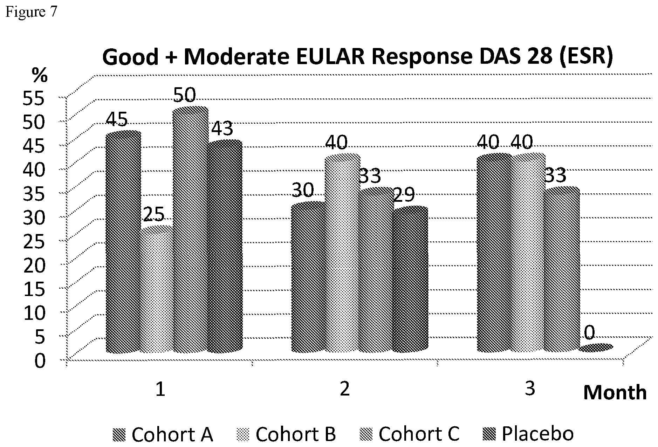

ACR20/50/70 responses were observed in 45/20/5% of patients receiving 1x10⁶ eASCs/kg versus 28/14/5% of patients on placebo at month 1. At month 3, ACR responses were 25/15/5 and 0/0/0 in cohort A and placebo respectively, which were maintained until month 6 (Figures 2-4). The DAS 28 (< 2.6) for the eASC treated group and placebo was 7% versus 0% at month 1 and 11% versus 0% at month 2 respectively (Figure 6). The DAS 28 (ESR) <3.2 results are shown in Figure 5. The results for good + moderate EULAR response DAS 28 (ESR) are shown in Figure 7.

Conclusion: These early clinical results are the first to suggest that IV infusion of eASCs is well tolerated in the treatment of refractory RA. Encouraging results include the absence of safety signals within 24 weeks and the larger improvement in secondary endpoints in the eASC arms versus placebo.

This is the first randomized trial with stem cells in refractory Rheumatoid Arthritis. A dose-limiting safety signal was not identified. Only one patient experienced a serious adverse event leading to discontinuation of the treatment. All other side effects were mild and transient: Most common related adverse events in eASCs group (3% or more reported): fever, headache, asthenia, respiratory tract infection, pruritus, malaise, rush and influenza-like illness (Table 5). No signs of hematological toxicity. Adverse events were evaluated by an independent Multidisciplinary Safety Board.

**Table 1**

| | **Cohort A** | **Cohort B** | **Cohort C** | **Placebo** |
|---|---|---|---|---|
| **DAS 28 (ESR), Median (range)** | 6,03 (4,1-8,4) | 5,62 (3,7-8,0) | 6,64 (3,6-7,3) | 5,88 (4,5-6,6) |
| **DAS 28 (CRP), Median (range)** | 5,57 (4,2-7,9) | 5,17 (3,2-7,2) | 5,88 (3,7-6,2) | 5,04 (3,6-5,9) |
| **SF-36 Physic Health, Median (range)** | 21,6 (5-56) | 22,5 (5-57) | 18,4 (6-24) | 15,0 (11-27) |
| **SF-36 Mental Health, Median (range)** | 31,2(7-61) | 45,5 (16-65) | 19,4 (9-65) | 29,4(12-60) |

**Table 1A**

| | Cohort A (N=20) | Cohort B (N=20) | Cohort C (N=6) | Placebo (N=7) |
|---|---|---|---|---|
| Gender, % female | 90 | 90 | 100 | 86 |
| Age, Median (range) years | 54.5 (37-68) | 58.0 (33-78) | 50 (33-77) | 64 (33-76) |
| Disease Duration (range) years | 13.8 (5-33) | 9.0(3-32) | 18.8 (11-32) | 15 (5-69) |
| Tender Joints, Median (range) | 25.0 (5-63) | 17.0 (3-62) | 20.5 (14-41) | 13.0 (6-20) |
| Swollen Joints, Median (range) | 12.5 (4-41) | 10.5 (4-36) | 11.0 (10-16) | 7.0(1-12) |
| ESR, Median (range) | 31.0 (2-96) | 31.0 (4-72) | 39.5 (11-115) | 39.0(23-50) |
| CRP, Median (range) | 0.93 (0.0-9.4) | 0.74(0.0-4.4) | 1.67 (0.0-4.3) | 0.50 (0.0-2.7) |
| HAQ score, Median (range) | 1.9 (0.5-3.0) | 1.5 (0.4-2.5) | 2.0 (1.0-2.5) | 2.0 (0.4-2.6) |
| DAS 28, Median (range) | 6.03 (4.1-8.4) | 5.62 (3.7-8.0) | 6.64 (3.6-7.3) | 5.88 (4.5-6.6) |

**Table 1B**

| No. of biologics | Cohort A (N=20) | Cohort B (N=20) | Cohort C (N=6) | Placebo (N=7) |
|---|---|---|---|---|
| 2 Biologics | 55% | 60% | 33.3% | 57.1% |
| 3 Biologics | 10% | 20% | 33.3% | 14.2% |
| Greater than 3 Biologics | 35% | 20% | 33.3% | 28.5% |

**Table 2**

| | **ITT (N=53)** |
|---|---|
| **Corticosteroids** | 46 (87%) |
| **NSAIDs** | 39 (74%) |
| **DMARDs** | 53 (100%) |
| **Biologics** | 53 (100%) |

**Table 3**

| **Previous biologics** | **Mechanism of action** | **ITT (N=53)** |
|---|---|---|
| Abatacept(Orencia) | Selective costimulation modulator | 4 (26%) |
| Adalimumab(Humira) | TNF-α inhibitor | 34 (64%) |
| Anakirna (Kineret) | IL-1 inhibitor | 1 (2%) |
| Certolizumab (Cimzia) | TNF-α inhibitor | 3 (6%) |
| Etanercept (Enbrel) | TNF-α inhibitor | 35 (66%) |
| Golimumab (Simponi) | TNF-α inhibitor | 3 (6%) |
| Infliximab (Remicade) | TNF-α inhibitor | 27 (51%) |
| Rituximab (Rituxan) | B-cell targetted therapies | 20 (38%) |
| Tocilizumab (Actembra) | IL-6 inhibitor | 18 (34%) |

**Table 4**

| **Previous DMARDs** | **ITT (N=53)** |
|---|---|
| Auranofin | 46 (87%) |
| Choroquine | 39 (74%) |
| Ciclosporin | 9 (17%) |
| Cyclophosphamide | 1 (2%) |
| Gold preparations | 16 (30%) |
| Hydroxychloroquin e sulfate | 12 (23%) |
| Leflunomide | 37 (70%) |
| Methotrexate | 53 (100%) |
| Penicillamine | 3 (6%) |
| Sodium Aurothiomalate | 6 (11%) |
| Sulfasalazine | 18 (34%) |

**Table 5**

| | 1x10⁶ eASCs/kg group (N=20) | 2x10⁶ eASCs/kg group (N=20) | 4x10⁶ eASCs/kg group (N=6) | Placebo group (N=7) |
|---|---|---|---|---|
| Pyrexia | 2 (10%) | 6 (30%) | 1 (16.7%) | 0 (0%) |
| Headache | 2 (10%) | 3 (15%) | 1 (16.7%) | 0 (0%) |
| Urinary tract infection | 3 (15%) | 1 (5%) | 2 (33.4%) | 0 (0%) |
| Upper respiratory tract infection | 3 (15%) | 1 (5%) | 1 (16.7%) | 0 (0%) |
| Respiratory tract infection | 3 (15%) | 0 (0%) | 0 (0%) | 0 (0%) |
| Nausea | 4 (20%) | 1 (5%) | 0 (0%) | 0 (0%) |
| Malaise | 1 (5%) | 2 (10%) | 0 (0%) | 0 (0%) |
| Vomiting | 2 (10%) | 1 (5%) | 0 (0%) | 0 (0%) |
| Asthenia | 1 (5%) | 1 (5%) | 0 (0%) | 1 (5%) |

## Claims

1. A composition comprising a substantially pure population of expanded mesenchymal stem cells (MSCs), wherein the MSCs are allogeneic adipose tissue derived stromal stem cells (ASCs), for use in a method of treating refractory rheumatoid arthritis in a human subject, wherein the composition is intravenously administered to the subject in repeated doses of about 1 million cells/kg at weekly intervals.

2. The composition for use according to any of the preceding claims, wherein at least about 50% of the MSCs express one or more of the markers CD9, CD10, CD13, CD29, CD44, CD49A, CD51, CD54, CD55, CD58, CD59, CD90 and CD105.

3. The composition for use according to any of the preceding claims, wherein the MSCs are expanded adipose tissue derived stromal stem cells, wherein at least about 50% of the MSCs express CD59 and/or CD90, for example wherein at least 65%, at least 70%, or 65-95%, or 70-90% of the MSCs express CD59 and/or CD90.

4. The composition for use according to claim 2 or claim 3, wherein at least about 50% of the MSCs do not express the markers CD11b, CD11c, CD14, CD31, CD34, CD45, CD133 and HLAII.

5. The composition for use according to any of the preceding claims, wherein the MSCs are **characterised in that** they:
a) do not express markers specific for antigen-presenting cells (APC);
b) do not express indoleamine 2,3-dioxygenase (IDO) constitutively; and
c) express IDO upon stimulation with interferon-gamma (IFN-γ).

6. The composition for use according to any of the preceding claims, wherein the MSCs are administered in a pharmaceutically acceptable carrier and/or a diluent.

7. The composition for use according to any of the preceding claims, wherein the MSCs are administered in conjunction with one or more further therapeutic agents.

8. The composition for use of claim 1, wherein the patient has refractory rheumatoid arthritis and is intravenously administered a dose of 1 million expanded Adipose-derived Mesenchymal Stem cells/kg on days 1, 8 and 15.

9. The composition for use of claim 8, wherein the patient is refractory to at least one or at least two biological treatments, for example any one of the biologicals listed in Table 3 such as a TNF-α inhibitor, optionally Adalimumab (Humira), Certolizumab (Cimzia), Etanercept (Enbrel), Golimumab (Simponi), or Infliximab (Remicade).

10. The composition for use of claim 1, wherein the subject is refractory to at least one biological treatment, optionally a TNF-α inhibitor, and is intravenously administered a dose of about 1 x 10⁶ cells/kg, at days 1, 8 and 15, wherein the dose comprises a population of cells comprising expanded adipose tissue derived stromal stem cells, wherein the majority of cells express CD59 and/or CD90, typically wherein expression of CD59 and/or CD90 within the population is greater than 50%, greater than 65%, or greater than 70%, e.g. 65-95%, or 70-90%, for treating rheumatoid arthritis in a subject.

## Patentansprüche

1. Zusammensetzung, umfassend eine im Wesentlichen reine Population von expandierten mesenchymalen Stammzellen (MSCs), wobei die MSCs aus allogenem Fettgewebe abgeleitete Stromastammzellen (ASCs) sind, für die Verwendung in einem Verfahren zur Behandlung von refraktärer rheumatoider Arthritis bei einem menschlichen Individuum, wobei die Zusammensetzung dem Individuum in wiederholten Dosen von etwa 1 Million Zellen/kg in wöchentlichen Intervallen intravenös verabreicht wird.

2. Zusammensetzung für die Verwendung gemäß einem der vorhergehenden Ansprüche, wobei mindestens etwa 50% der MSCs einen oder mehrere der Marker CD9, CD10, CD13, CD29, CD44, CD49A, CD51, CD54, CD55, CD58, CD59, CD90 und CD105 exprimieren.

3. Zusammensetzung für die Verwendung gemäß einem der vorhergehenden Ansprüche, wobei die MSCs expandierte Fettgewebe-abgeleitete Stromastammzellen sind, wobei mindestens etwa 50% der MSCs CD59 und/oder CD90 exprimieren, wobei zum Beispiel mindestens 65%, mindestens 70% oder 65-95% oder 70-90% der MSCs CD59 und/oder CD90 exprimieren.

4. Zusammensetzung für die Verwendung gemäß Anspruch 2 oder Anspruch 3, wobei mindestens etwa 50% der MSCs die Marker CD11b, CD11c, CD14, CD31, CD34, CD45, CD133 und HLAII nicht exprimieren.

5. Zusammensetzung für die Verwendung gemäß einem der vorhergehenden Ansprüche, wobei die MSCs **dadurch gekennzeichnet sind, dass** sie:
a) keine Marker exprimieren, die für antigenpräsentierende Zellen (APC) spezifisch sind;
b) Indolamin-2,3-Dioxygenase (IDO) nicht konstitutiv exprimieren; und
c) IDO nach Stimulation mit Interferon-gamma (IFN-γ) exprimieren.

6. Zusammensetzung für die Verwendung gemäß einem der vorhergehenden Ansprüche, wobei die MSCs in einem pharmazeutisch akzeptablen Träger und/oder einem Verdünnungsmittel verabreicht werden.

7. Zusammensetzung für die Verwendung gemäß einem der vorhergehenden Ansprüche, wobei die MSCs zusammen mit einem oder mehreren weiteren therapeutischen Mitteln verabreicht werden.

8. Zusammensetzung für die Verwendung von Anspruch 1, wobei der Patient eine refraktäre rheumatoide Arthritis hat und ihm intravenös eine Dosis von 1 Million expandierten Fett-abgeleiteten mesenchymalen Stammzellen/kg an den Tagen 1, 8 und 15 verabreicht wird.

9. Zusammensetzung für die Verwendung von Anspruch 8, wobei der Patient gegenüber mindestens einer oder mindestens zwei Biologika-Behandlungen, beispielsweise irgendeinem der in Tabelle 3 aufgelisteten Biologika, wie einem TNF-α-Inhibitor, gegebenenfalls Adalimumab (Humira), Certolizumab (Cimzia), Etanercept (Enbrel), Golimumab (Simponi) oder Infliximab (Remicade), refraktär ist.

10. Zusammensetzung für die Verwendung von Anspruch 1, wobei das Individuum gegenüber mindestens einer Biologika-Behandlung, gegebenenfalls einem TNF-α-Inhibitor, refraktär ist und ihm intravenös eine Dosis von etwa 1 x 10⁶ Zellen/kg an den Tagen 1, 8 und 15 verabreicht wird, wobei die Dosis eine Population von Zellen umfasst, umfassend expandierte Fettgewebe-abgeleitete Stromastammzellen, wobei die Mehrheit der Zellen CD59 und/oder CD90 exprimiert, wobei typischerweise die Expression von CD59 und/oder CD90 innerhalb der Population bei mehr als 50%, mehr als 65% oder mehr als 70%, z. B. 65-95% oder 70-90%, vorliegt, zur Behandlung von rheumatoider Arthritis in einem Individuum.

## Revendications

1. Composition comprenant une population sensiblement pure de cellules souches mésenchymateuses expansées (CSM), dans laquelle les CSM sont des cellules stromales dérivées de tissu adipeux allogéniques (CSA), pour utilisation dans un procédé de traitement de la polyarthrite rhumatoïde réfractaire chez un sujet humain, la composition étant administrée par voie intraveineuse au sujet sous forme de doses répétées d'environ 1 million de cellules/kg à des intervalles hebdomadaires.

2. Composition pour utilisation selon l'une quelconque des revendications précédentes, dans laquelle au moins environ 50 % des CSM expriment l'un ou plusieurs des marqueurs CD9, CD10, CD13, CD29, CD44, CD49A, CD51, CD54, CD55, CD58, CD59, CD90 et CD105.

3. Composition pour utilisation selon l'une quelconque des revendications précédentes, dans laquelle les CSM sont des cellules souches stromales dérivées de tissu adipeux expansées, dans laquelle au moins environ 50 % des CSM expriment CD59 et/ou CD90, par exemple dans laquelle au moins 65 %, au moins 70 %, ou 65 à 95 %, ou 70 à 90 % des CSM expriment CD59 et/ou CD90.

4. Composition pour utilisation selon la revendication 2 ou la revendication 3, dans laquelle au moins environ 50 % des CSM n'expriment pas les marqueurs CD11b, CD11c, CD14, CD31, CD34, CD45, CD133 et HLAII.

5. Composition pour utilisation selon l'une quelconque des revendications précédentes, dans laquelle les CSM sont **caractérisées en ce qu'**elles :
a) n'expriment pas des marqueurs spécifiques pour des cellules présentatrices d'antigène (CPA) ;
b) n'expriment pas l'indoleamine 2,3-dioxygénase (IDO) constitutivement ; et
c) expriment IDO après stimulation avec l'interféron-gamma (IFN-γ).

6. Composition pour utilisation selon l'une quelconque des revendications précédentes, dans laquelle les CSM sont administrées dans un véhicule et/ou un diluant pharmaceutiquement acceptable.

7. Composition pour utilisation selon l'une quelconque des revendications précédentes, les CSM étant administrées conjointement avec un ou plusieurs agents thérapeutiques supplémentaires.

8. Composition pour utilisation selon la revendication 1, le patient étant atteint de polyarthrite rhumatoïde réfractaire et il est administré à celui-ci par voie intraveineuse une dose de 1 million de cellules souches mésenchymateuses d'origine adipeuse expansées/kg aux jours 1, 8 et 15.

9. Composition pour utilisation selon la revendication 8, le patient étant réfractaire à au moins un ou au moins deux traitements biologiques, par exemple l'un quelconque des agents biologiques répertoriés dans le tableau 3, tels qu'un inhibiteur de TNF-α, éventuellement l'adalimumab (Humira), le certolizumab (Cimzia), l'étanercept (Enbrel), le golimumab (Simponi), ou l'infliximab (Remicade).

10. Composition pour utilisation selon la revendication 1, le sujet étant réfractaire à au moins un traitement biologique, facultativement un inhibiteur de TNF-α, et il est administrée par voie intraveineuse à celui-ci une dose d'environ 1 x 10⁶ cellules/kg, aux jours 1, 8 et 15, la dose comprenant une population de cellules souches stromales dérivées de tissu adipeux expansées, où la majorité des cellules expriment CD59 et/ou CD90, typiquement où l'expression de CD59 et/ou CD90 dans la population est supérieure à 50 %, supérieure à 65 %, ou supérieure à 70 %, par exemple 65 à 95 %, ou 70 à 90 %, pour traiter la polyarthrite rhumatoïde chez un sujet.
